# EUROPÄISCHE PATENTSCHRIFT

(11) **EP 1 738 679 B1**
(45) Veröffentlichungstag und Bekanntmachung des Hinweises auf die Patenterteilung: **07.12.2011**
(21) Anmeldenummer: 06113938.2
(22) Anmeldetag: 15.05.2006
(51) Int. Cl.: H05K 7/20

(54) **Endoskop mit einer Kühleinrichtung**
Endoscope with cooling device
Endoscope avec un dispositif de refroisissement

(30) Priorität: 01.07.2005 DE 102005030861
(43) Veröffentlichungstag der Anmeldung: 03.01.2007
(73) Patentinhaber: INVENDO MEDICAL GMBH, 69469 Weinheim (DE)
(72) Erfinder: Bob, Dr. Konstantin, 69469, Weinheim (DE); Pauker, Fritz, 86438, Kissing (DE); Viebach, Thomas, 86579, Diepoltshofen (DE)
(74) Vertreter: Winter, Brandl, Fürniss, Hübner, Röss, Kaiser, Polte - Partnerschaft

(56) Entgegenhaltungen:
- EP-A- 1 507 449
- JP-A- 2002 177 197
- JP-A- 2005 027 851
- US-A- 5 825 642
- US-A1- 2002 091 468
- US-A1- 2003 147 214
- US-A1- 2004 064 018
- US-A1- 2004 248 059
- US-A1- 2005 075 538

## Beschreibung

Die vorliegende Erfindung betrifft ein Endoskop mit einer Kühleinrichtung für elektronische Bauteile.

Elektronische Bauteile wie lichtempfindliche Sensoren, lichtemittierende Dioden (LED's) usw., werden in zunehmendem Masse in medizinischen Vorrichtungen, bspw. Endoskopievorrichtungen, eingesetzt. Endoskope haben an ihrem distalen Ende in der Regel eine visuelle Einrichtung in Form eines lichtempfindlichen Mikrochips, einer der vorgeschalteten Optik sowie einer Beleuchtungseinrichtung. Insbesondere bei derartigen Instrumenten ist es erforderlich, die durch die Beleuchtungseinrichtungen sowie die Körpertemperatur selbst entstehende Wärme durch geeignete Kühleinrichtungen zu kompensieren oder die lichtempfindlichen elektronischen Bauteile vor Überhitzung durch eine entsprechende Isolation zu schützen.

Aus der DE 196 26 433 A1 ist daher ein Endoskopkopf für ein für diagnostische sowie therapeutische Zwecke in Körperhohlräumen geeignetes Endoskop mit Beleuchtungseinrichtung, einer Optik sowie einer Bilderfassungseinrichtung bekannt geworden, das an seiner distalen Stirnseite angeordnete lichtemittierende Elemente sowie eine integrierte Bilderzeugungseinrichtung hinter einer Endoskopoptik aufweist. Des Weiteren ist gemäß diesem Stand der Technik eine Kühleinrichtung vorgesehen zum Hindurchleiten eines Kühlfluids, wobei das Kühlfluid an der Bilderzeugungseinrichtung vorbeigeführt wird. Hierdurch wird erreicht, dass die wärmempfindliche Bilderfassungseinrichtung vor einer Überhitzung geschützt wird, um hierdurch die Bildqualität insgesamt zu verbessern.

Es hat sich jedoch gezeigt, dass sich die Kühlung der Bilderzeugungseinrichtung selbst hinsichtlich der Gesamtfunktion eines Endoskopkopfs dieser Gattung als unzureichend erweist. Wie eingangs bereits ausgeführt wurde, sind derartige Endoskopköpfe mit einer Mehrzahl von elektronischen sowie ggf. hydraulischen/pneumatischen Einrichtungen bestückt, welche keinen bzw. nur geringen thermodynamischen Belastungen ausgesetzt werden dürfen. Insbesondere die Scharfstellvorrichtung der Endoskopoptik ist besonders wärmeempfindlich, da bei Temperaturerhöhung keine optimale Justierung der optischen Elemente mehr möglich ist.

Aus der US 2005/0075538 A1 ist zur Lösung dieses Problems ein Endoskop der Ein-Weg-Bauart bekannt geworden, das ebenfalls eine Kühleinrichtung für elektronische Bauteile benutzt, diese Kühleinrichtung jedoch den LED's zuordnet. In anderen Worten ausgedrückt, hat sind die LED's als Bestandteil der Kühleinrichtung ausgebildet, welche so ausgestaltet ist, dass sie in eine vordere Kappe des Endoskops eingepasst werden kann, in welcher auch die anderen Endoskop spezifischen Bauteile wie Optik, Sprüheinrichtung u. dgl. untergebracht sind.

Die Kühleinrichtung ist hierfür in Form einer halbzylindrischen Manschette ausgebildet, in der Steckanschlüsse für die LED's integriert sind, und welche an ihrer Stirnseite Fassungen zur Aufnahme der LED's aufweist. Darüber hinaus wird hierdurch die gesamte Optik einschliesslich dem Linsensystem sowie dem lichtempfindlichen Sensor von der Kühleinrichtung umschlossen und somit von den LED's thermisch abgeschirmt.

Die Erfinderin der vorliegenden Erfindung hat jedoch festgestellt, dass durch diese besondere Ausgestaltung der Kühleinrichtung der ohnehin bei Endoskopen äußerst beschränkte Bauraum weiter reduziert wird. Darüber hinaus sind die LED's gemäß diesem Stand der Technik trotz Kühlung immer noch in der Lage je nach Ausrichtung mit Gewebe der zu untersuchenden Körperhöhle in Kontakt zu kommen und ggf. sogar Verbrennungen zu verursachen.

Als weiterer Stand der Technik sei noch auf die JP 2005 027851 A verwiesen, die ein Endoskop mit einer Endkappe, welche wärmeerzeugende elektrische Bauelemente sowie eine Optik umfasst, und mit einer einen Kühlkreislauf sowie eine Kühlkammer umfassenden Kühleinrichtung zur Kühlung der Bauelemente offenbart.

Zwischen dem lichtempfindlichen Sensor und den Beleuchtungskörpern ist ein Wärmeisolationsbauteil angeordnet, das den Sensor vor Überhitzung durch die Beleuchtungskörper schützen soll. Beleuchtungskörpern ist ein Wärmeisolationsbauteil angeordnet, das den Sensor vor Überhitzung durch die Beleuchtungskörper schützen soll.

Es ist daher die Aufgabe der vorliegenden Erfindung, eine gattungsgemässe Kühleinrichtung für elektronische Bauteile eines Endoskops, insbesondere bei der Verwendung in medizinischen Einrichtungen, zu schaffen, die eine höhere Effizienz insbesondere auch zur Vermeidung von Gewebeschäden aufweist.

Diese Aufgabe wird durch ein gattungsgemäßes Endoskop mit einer Kühleinrichtung mit den Merkmalen gemäß dem anliegenden Patentanspruch 1 gelöst.

Der Kerngedanke der Erfindung besteht im wesentlichen darin, im Gegensatz zum Stand der Technik nicht die wärmeempfindlichen Bauteile des Endoskops vor Wärmebelastung zur schützen, sondern Wärmeenergie an jenen Stellen zu vernichten, an welchen Wärme erzeugt wird. Entscheidend hierbei ist, dass die Kühleinrichtung bezüglich der zu kühlenden elektrischen Bauteile in einer Richtung angeordnet ist, in der insbesondere bei Endoskopen noch ausreichend Bauraum zur Verfügung steht, d.h. in Endoskoprichtung gesehen hinter den wärmeerzeugenden elektrischen Bauteilen bzw. auf der Rückseite der die wärmeerzeugenden elektrischen Bauteile tragenden Platine.

Erfindungsgemäß ist es daher vorgesehen, die endoskopeigene Optik, Beleuchtungsmittel wie LED's usw. in der die Endoskopspitze bildenden Kappe unterzubringen, wohingegen die Kühleinrichtung zur Kappe baulich getrennt in dem vorgeschalteten sogenannten Deflecting, d.h. dem abkrümmbaren Teil des Endoskopschafts angeordnet ist.

Die Beleuchtungsmittel sind dabei in konventioneller Weise auf einer Platine aufgelötet, welche den unteren Abschluss der Endoskopkappe bildet und welche an ihrer Unterseite vorzugsweise eine Anlagefläche aufweist. Darüber hinaus ragen vorzugsweise die Anlötfüsschen der Beleuchtungsmittel und/oder zusätzliche Kontaktstifte über die Unterseite der Platine vor.

Die Kühleinrichtung bildet vorzugsweise die äußerste Stirnseite des Deflecting und hat eine wärmeleitende Abdeckung, auf deren Unterseite sich ein erster Wärmetauscher befindet, und die bei der Montage des Endoskops in thermischen Kontakt mit der Platine bringbar ist.

Weiterhin sieht die Kühleinrichtung einen Kühlkreislauf vor, bestehend aus einer Kühlfluidpumpe, dem ersten Wärmetauscher zur Aufnahme und Abfuhr von Wärmeenergie an einem Wärme erzeugenden elektronischen Bauteil, einem zweiten Wärmetauscher zur Abgabe der aufgenommenen Wärmeenergie an die Atmosphäre sowie einer Regeleinrichtung bestehend aus einer Wärmeenergieerfassungseinheit zum Erfassen der von dem Wärme erzeugenden elektronischen Bauteil abgegebenen Wärmemenge sowie einer Einstelleinheit zum Einstellen eines Fluidstroms in Abhängigkeit der erfassten Wärmeenergie derart, dass sich bei einer bestimmten Temperatur ein Gleichgewicht zwischen erzeugter Wärmemenge und abgeführter Wärmemenge einstellt.

Auch ist es erfindungsgemäß vorgesehen, jedes in der Kappe des Endoskops ungebrachten Beleuchtungsmittel vorzugsweise LED's in jeweils einen Reflektor einzusetzen, der die Form eines Kelchs mit einem vorzugsweise parabelförmigen Längsschnitt hat, und der die Strecke zwischen der die Rückwand der Kappe bildenden Platine, auf der die Beleuchtungsmittel angeordnet sind, und der Vorderseite der Kappe überbrückt.

Weitere vorteilhafte Ausgestaltungen der Erfindung sind dabei Gegenstand der übrigen Unteransprüche.

Die Erfindung wird nachstehend anhand bevorzugter Ausführungsbeispiele unter Bezugnahme auf die begleitenden Figuren näher erläutert.
Fig. 1 zeigt eine Prinzipdarstellung einer Kühlungseinrichtung
Fig. 2 zeigt die Prinzipdarstellung einer elektronischen Leiterplatine als Bestandteil der Kühleinrichtung gemäß Fig. 1
Fig. 3 zeigt den prinzipiellen Seitenriss der elektronischen Leiterplatte und Kühleinrichtung gemäß der Fig. 2, und
Fig. 4 zeigt den distalen Endbereich eines Endoskops mit einer Kühleinrichtung gemäß einem bevorzugten Ausführungsbeispiel der Erfindung.

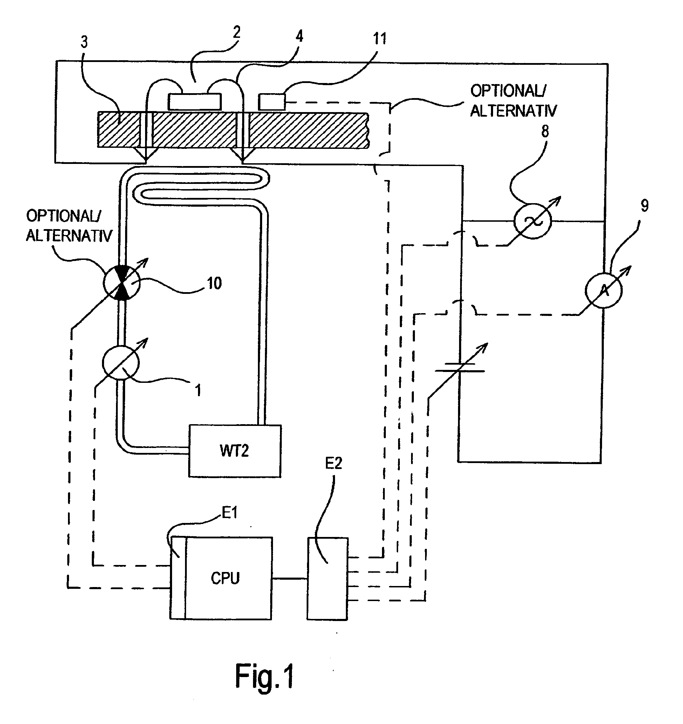

Gemäß der Prinzipdarstellung in Fig. 1 hat die Kühleinrichtung grundsätzlich einen Kühlkreislauf zur Kühlung eines Wärme erzeugenden elektronischen Bauteils beispielsweise eines Beleuchtungsmittels vorzugsweise LED sowie eine Regeleinrichtung zur Einstellung des Fluidstroms in Abhängigkeit der aktuell erzeugten Wärmemenge. Alternativ hierzu ist es natürlich auch möglich, die erzeugte Wärmemenge bauteilspezifisch sowie entsprechend der zu erwartenden Betriebsbedingungen vorab zu ermitteln und die Fördermenge an Kühlfluid entsprechend der abgeschätzten, voraussichtlich erzeugten Wärmemenge voreinzustellen.

Der Kühlkreislauf umfasst eine Förderpumpe 1, einen ersten Wärmetauscher WT1, der über einen Förderkanal mit der Förderpumpe 1 fluidverbunden ist, sowie einen zweiten Wärmetauscher WT2, der mit dem ersten Wärmetauscher WT1 sowie der Förderpumpe 1 fluidverbunden ist (geschlossener Kreislauf). Alternativ hierzu ist es auch möglich, den zweiten Wärmetauscher WT2 wegzulassen und statt dessen das verbrauchte, d.h. aufgeheizte Kühlfluid direkt zu entsorgen (offener Kreislauf).

Wie aus der Prinzipskizze gemäß der Fig. 1 ferner zu entnehmen ist, befindet sich der erste Wärmetauscher WT1 unmittelbar im Bereich eines Wärme erzeugenden elektrischen Bauteils 2, bspw. eines Beleuchtungsmittels wie ein LED oder eines Leistungstransistors, welches auf eine elektrische Leiterplatte (Platine) 3 gelötet/gesteckt ist.

Versuche haben gezeigt, dass bis zu 80% der Wärmeenergie, welche von einem elektronischen Bauteil 2 wie einer LED erzeugt wird, über die elektrischen Anschlussfüsse 4 vorzugsweise an die Leiterplatine 3 abgegeben wird. Aus diesem Grund erweist es sich als besonders effektiv, den Wärmetauscher WT1 unterhalb der Leiterplatine 3 im Bereich der Anlötpunkte der Leiterfüsschen 4 des Wärme erzeugenden elektrischen Bauteils 2 zu platzieren.

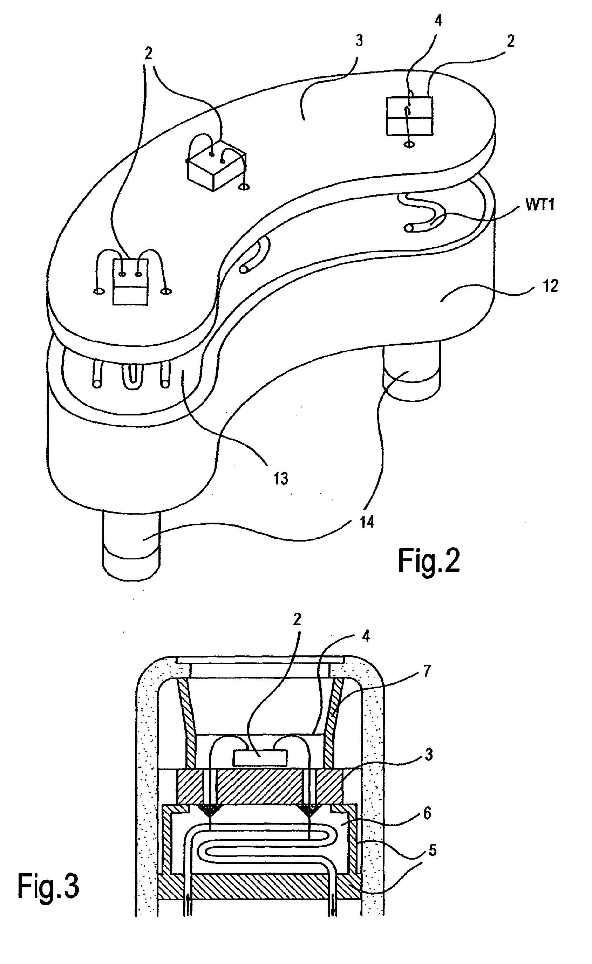

In der Fig. 3 ist eine prinzipielle Anordnung des Wärmetauschers WT1 unterhalb eines Wärme erzeugenden elektrischen Bauteils 2 dargestellt.

Gemäß der Fig. 3 ruht die Platine 3 auf einem Sockelelement 5, welches unterhalb der Platine 3 einen Kühlraum 6 ausbildet.

Wie in der Fig. 3 ferner vergrößert dargestellt ist, ragen die Anschlussfüsschen 4 des elektrischen Bauteils 2 abschnittsweise in den Kühlraum 6, um so Wärme an den durch den Kühlraum 6 geführten Wärmetauscher WT1 abzugeben. Der Wärmetauscher WT1 ist in Fig. 3 als Kühlschlange in geschlossener Bauweise dargestellt. Indessen ist es jedoch auch möglich, den Kühlraum 6 dichtend durch die Platine 2 selbst oder durch eine mit der Platine in Anlage bringbare Wärmetransferplatte zu verschließen und das Kühlfluid in offener Bauweise direkt in den Kühlraum 6 einzuleiten.

Das elektrische Bauteil 2 ist gemäß der Fig. 3 als ein Beleuchtungsmittel vorzugsweise ein LED ausgebildet, welches zur Verstärkung der Lichtausbeute von einem Reflektor 7, vorzugsweise aus Aluminium, trichterförmig umgeben ist.

Aluminium ist bekanntermaßen ein hervorragender Wärmeleiter.

Da, wie vorstehend bereits ausgeführt wurde, zumindest noch 20% der gesamten erzeugten Wärmemenge über das elektrische Bauteil 2 selbst an die Umgebung abgegeben wird, besteht durchaus die Möglichkeit, dass sich der Reflektor 7 erwärmt. Aus diesem Grund ist es optional möglich, den Wärmetauscher WT1 zusätzlich zumindest abschnittsweise um den Reflektor 7 herum zu führen, um die dort aufgenommene Wärmemenge über das hindurchgeleitete Kühlfluid abzuführen.

Die vorliegende Kühleinrichtung für elektronische Bauteile findet vorzugsweise in medizinischen Einrichtungen, wie insbesondere Endoskope, Verwendung. Endoskope sind in der Regel mit Endoskopköpfen ausgestattet, in denen eine Mehrzahl von elektrischen, optischen sowie hydraulischen Bauteilen auf engstem Raum angeordnet sind und sich demzufolge mechanisch wie auch thermodynamisch beeinflussen. In jüngster Zeit werden Endoskope mit lichtempfindlichen Mikrochips versehen, denen eine Optik vorgeschaltet ist, um so visuelle Bildsignale über ein Leitungssystem an einen Rechner zu übertragen. Derartige lichtempfindliche Mikrochips sowie die vorgeschalteten Optiken sind äußerst wärmeempfindlich und müssen daher insbesondere bei Verwendung innerhalb eines Körperhohlraums vor Überhitzung geschützt werden.

Um optimale Bildsignale zu erhalten, ist eine zusätzliche Beleuchtungseinrichtung in Form des zuvor beschriebenen LED erforderlich, welches trotz hohen Wirkungsgrades an Lichtausbeute immer noch eine nicht unerhebliche Wärmeenergie erzeugt. Ferner ist die Versorgung des Endoskopkopfs mit elektrischer Energie und/oder einem hydraulischen/pneumatischen Fluid aufgrund der räumlichen Enge grundsätzlich problematisch. D.h., die Durchmesser von Zu- und Abfuhrkanälen können nicht beliebig gross gestaltet werden, sondern sind an die räumlichen Begebenheiten kompromisslos anzupassen.

Aus diesem Grund ist es vorgesehen, die von einem elektrischen Bauteil 2, wie dem LED, erzeugte Wärmemenge zu erfassen und die Menge an Kühlfluid, welche zur Kühlung des Wärme erzeugenden elektrischen Bauteils 2 erforderlich ist, der erfassten Wärmemenge anzupassen. Auf diese Weise lässt sich ein optimaler Wirkungsgrad der gesamten Einrichtung erreichen und den Energieverbrauch der Pumpe 1 bzw. den Querschnitt der Fluidkanäle des Kühlkreislaufs optimieren.

Gemäß der Fig. 1 ist es hierfür vorgesehen, dem Wärme erzeugenden elektrischen Bauteil 2 einen elektrischen Messkreis beizuordnen. Dieser besteht aus einem Spannungs-8 und einem Strommessgerät 9, deren aktuell erfasste Werte einer Recheneinheit (CPU) zugeführt werden.

In der CPU sind bauteilspezifische Wertetabellen bezüglich Spannung, Strom und Wärmeenergie abgespeichert. Derartige Tabellen können bauteilspezifisch vorab analytisch erstellt werden. Mit anderen Worten ausgedrückt erzeugen elektrische Bauteile bei bestimmten Strom-Spannungs-Verhältnissen eine vorbestimmte Wärmeenergie, welche tabellarisch erfasst werden kann. Die CPU vergleicht nunmehr die aus den gemessenen Strom-/Spannungswerten erhaltene Wärmeenergie mit der aktuellen Fördermenge an Kühlfluid durch die Pumpe 1 und steuert diese entsprechend an.

Anders ausgedrückt lassen sich für einen vorbestimmten Kanalquerschnitt sowie Wärmetauscher spezifische Wärmemengen analytisch bestimmen, welche bei einer bestimmten Förderleistung der Pumpe 1 maximal abgeführt werden können. Die CPU regelt daher die Pumpe 1 derart, dass deren Förderleistung sowie die hiervon geförderte Menge an Kühlfluid gerade ausreicht, um die errechnete Wärmemenge, welche vom betreffenden elektronischen Bauteil erzeugt wird, zu absorbieren.

Alternativ oder optional ist es natürlich auch möglich, anstelle der bzw. zusätzlich zur regelbaren Pumpe 1 ein regelbares Drosselventil 10 der Pumpe 1 nachzuschalten, um hierdurch die Fördermenge an Kühlfluid in Abhängigkeit der errechneten Wärmeenergie zu regeln bzw. zu steuern. Auch ist es möglich, die dem elektronischen Bauteil 2 zugeführte elektrische Energie bei Überschreiten einer vorbestimmten erzeugten Wärmemenge zu reduzieren, insbesondere in dem Fall, dass die maximale Kühlleistung der Kühleinrichtung unzureichend wird.

Wie in der Fig. 1 ferner dargestellt ist, kann zusätzlich oder alternativ zu dem dargestellten elektrischen Regelkreis ein Wärmesensor 11 vorgesehen sein, der in unmittelbarer Nähe zu dem Wärme erzeugenden elektrischen Bauteil 2 angeordnet ist und die aktuellen Temperaturwerte der CPU eingibt. Die Ansteuerung der regelbaren Pumpe 1 und/oder des nachgeschalteten Regelventils 10 erfolgt dementsprechend über eine Regeleinheit E1 der CPU.

In der Fig. 2 ist ein erstes Beispiel einer konstruktiven Ausgestaltung der Kühleinrichtung, insbesondere im Bereich der Platine 3 und des ersten Wärmetauschers WT1, dargestellt. Diese Einrichtung wird vorzugsweise im Bereich des distalen Endabschnitts eines nicht weiter dargestellten Endoskops verwendet.

Gemäß der Fig. 2 ist demzufolge die dort dargestellte Platine 3 teilkreisförmig ausgestaltet und trägt vorliegend drei lichtemittierende Dioden (LEDs) 2. Die Leiterplatine 3 ist auf ein Sockelelement 12 aufgeklebt, dessen äußere Form der Form der Leiterplatine 3 angeglichen ist, d.h. ebenfalls teilkreisförmig ausgestaltet ist.

Das Sockelelement 12 bildet eine trogförmige Ausnehmung 13, welche an der Oberseite des Sockelelements 12 von der Leiterplatte 3 bei deren Aufkleben verschlossen wird. Innerhalb der trogförmigen Ausnehmung 13 ist der Wärmetauscher WT1, gemäß der Fig. 2 durch drei Wärmeschlangen dargestellt, untergebracht. Schließlich sind an der der Leiterplatine 3 gegenüberliegenden Unterseite des Sockelelements 12 zwei jeweils endseitig angeordnete Anschlussstutzen 14 vorgesehen, über die die Leiterplatte 3 mit elektrischer Energie und der Wärmetauscher WT1 mit Kühlfluid versorgt werden.

Abschließend sei darauf hingewiesen, dass das Sockelelement 12 gemäß der Fig. 2 dem Sockel 5 gemäß der Fig. 3 funktionell entspricht. Es ist daher grundsätzlich möglich, die trogförmige Ausnehmung 13 dichtend auszubilden und direkt mit Kühlfluid zu fluten. Das Sockelelement 12 einschließlich der daran fixierten Beleuchtungsmittel/Platine ist im Fall des Einbaus in dem Endoskop vorzugsweise im Deflecting unterhalb der die Endoskopspitze bildenden Kappe angeordnet, wobei der in Fig. 3 dargestelle Reflektor 7 die Distanz zwischen Beleuchtungskörper und Lichtaustrittskante an der Stirnseite der nicht weiter dargestellten Kappe überbrückt.

Durch diese Maßnahme wird gewährleistet, dass der begrenzte Bauraum innerhalb der Kappe zur Aufnahme von Optik, Fluidkanälen, Sprüheinrichtungen usw. nicht weiter eingeengt wird, zum anderen wird hierdurch verhindert, dass das Beleuchtungsmittel zu nah an das umliegende Gewebe des zu untersuchenden Körperhohlraums kommt oder dieses sogar berührt und dabei Verbrennungen bewirkt.

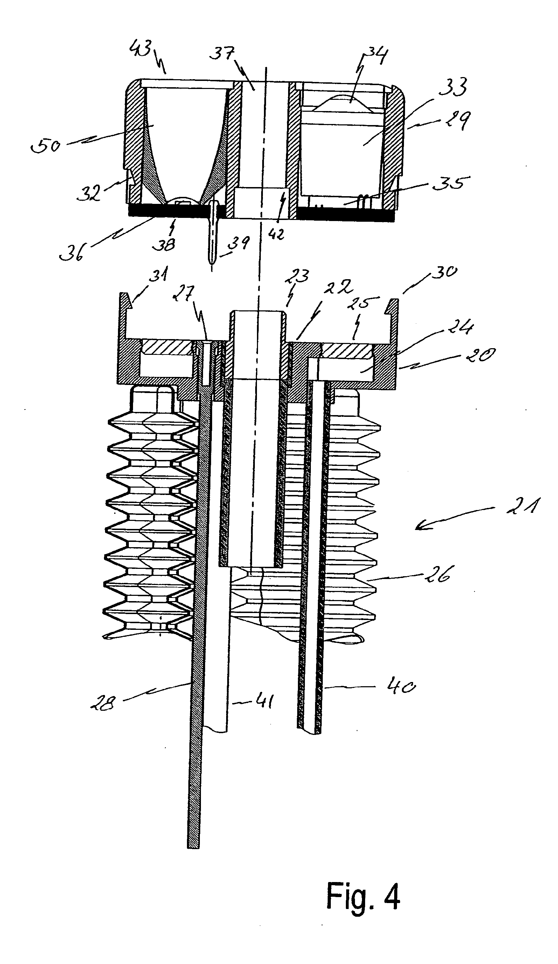

In der Fig. 4 ist ein bevorzugtes Ausführungsbeispiel der Erfindung gezeigt.

Demzufolge bildet die Kühleinrichtung die Endplatte 20 eines abkrümmbaren Zwischenstücks 21 des Schafts des erfindungsgemäßen Endoskops (nachfolgend als Deflecting bezeichnet), welche becher- bzw. napfförmig ausgestaltet ist. Der Becher 20 weist eine zentrale Durchgangsbohrung 22 auf, in der ein rohrstutzenförmigen Vorsprung 23 des Bechers eingesetzt ist. Hierdurch entsteht eine ringartige Rinne, welche eine Kühlkammer 24 der Kühleinrichtung darstellt.

Die offene Stirnseite der becherförmigen Endplatte 20 ist durch eine Ringscheibe oder Abdeckplatte 25 abgedeckt, welche aus einem wärmeleitenden Material besteht. Die Unterseite der Endplatte 20 bildet eine Anschlagsfläche für Betätigungselemente 26 des Deflecting wie hydraulisch/pneumatisch aktivierbare Faltenbalge, Piezoelemente, Zug-/Druckkabel und dgl. wie sie bereits aus dem Stand der Technik bekannt sind. Darüber hinaus ist in der Endplatte 20 eine Anzahl elektrischer Steckanschlüsse 27 ausgebildet, die über elektrische Kabel 28 mit einer Steuereinheit (nicht weiter dargestellt) verbunden sind.

Die Endplatte 20 bildet an ihrer äußeren Stirnseite ferner einen Anschluss für eine Endkappe 29 des Endoskops. Im vorliegenden Fall besteht dieser Anschluss aus einer Anzahl von Rastelementen in Form von Laschen 30, die randseitig an der Endplatte 20 angeordnet sind und sich von dieser axial erstrecken. Jede Lasche 30 ist endseitig mit einer Rastnase 31 ausgebildet, welche mit einer entsprechenden kappenseitigen Hinterschneidung 32 in Rasteingriff bringbar ist.

In der Endkappe 29 befinden sich eine Vielzahl von Bauteilen, wie beispielweise eine Optik 33 bestehend aus einem Linsensystem 34 und einem lichtempfindlichen Sensor 35, Beleuchtungsmittel 36, vorzugsweise LED's, ein zentraler Durchgangskanal für das Hindurchführen von medizinischen Instrumenten, usw.

Wie in der Fig. 4 gezeigt ist, sind zumindest die Beleuchtungsmittel 36 auf einer Platine 38 angeordnet, welche einen unteren Abschlussdeckel der Endkappe 29 bildet. An der Unterseite der Platine 38 ragen eine Anzahl von Anschlussstiften 39 vor, welche bei der Montage der Kappe 29 auf der Endplatte 20 des Deflecting in deren elektrische Steckanschlüsse 27 (Anschlussbuchsen) eingedrückt werden. Für eine passgenaue Montage der Endkappe 29 ragt der zentrale stutzenförmige Vorsprung 23 der becherförmigen Endplatte 20 über die wärmeleitende Abdeckplatte 25 axial vor, um als Zentrierhilfe in den Arbeitskanal 37 der Endkappe 29 eingepasst werden zu können. Hierbei wird die Endkappe 29 an ihrer unteren Stirnseite insbesondere über die unterseitige Platine 38, auf die Endplatte 20 des Deflecting aufgesetzt und zusätzlich zu der Haltekraft der Rastelemente 30 beispielsweise durch Verkleben fixiert. In Montageposition liegt die wärmeleitende Abdeckplatte 25 an der Unterseite der die Beleuchtungsmittel tragenden Platine 38 an, derart, dass ein Wärmetransfer zwischen Platine 38 und Endplatte stattfinden kann.

Gemäß der Fig. 4 sind durch das Deflecting zumindest zwei weitere Fluidkanäle 40, 41 geführt, von denen der Eine 40 ein mit der Kühlkammer fluidverbundener Zuführkanal und der Andere 41 ein Ablaufkanal bildet.

Alternativ zu der vorstehend beschriebenen Konstruktion ist es beim zweiten Ausführungsbeispiel gemäß der Fig. 4 auch möglich, auf die Abdeckplatte 25 zu verzichten und statt dessen die Kühlkammer 24 in offener Ausführung bei der Montage der Kappe 29 auf der Endplatte 20 unmittelbar von der Platine 38 selbst abdecken zu lassen. In diesem letzteren Fall könnte an der stirnseiten Mantelfläche der Endplatte 20 sowie an der Stirnseite des zentralen Vorsprungs 23 der becherförmigen Endplatte 20 jeweils eine Ringdichtung (nicht weiter gezeigt) vorgesehen sein, die sich beim Montieren der Endkappe 29 auf dem Deflecting 21 an Stirnseite der Endkappe 29 sowie an einem Wellenabsatz 42 des zentralen Arbeitskanals 37 der Kappe 29 anlegt und somit die Kühlkammer 24 radial abdichtet.

Schließlich ist in Fig. 4 ein Reflektor 50 dargestellt.

Wie vorstehend bereits ausgeführt wurde, wird durch das axiale Zurückziehen der Beleuchtungsmittel 36 sowie durch das Verlegen der Kühleinrichtung aus der Endkappe 29 in das unmittelbar dahinterliegende Deflecting 21 erreicht, dass innerhalb der Endkappe 29 des Endoskops ausreichender Bauraum für die anderen notwendigen Bauteile verbleibt. Darüber hinaus wird ein direkter Kontakt des Beleuchtungsmittels 36 mit umgebendem Gewebe verhindert. Um dennoch eine ausreichende Beleuchtung der Umgebung zu garantieren, ist zwischen dem Beleuchtungsmittel 36 vorzugsweise in Form eines LED und der vordersten Lichtaustrittskante, welche durch eine Scheibe/Fenster 43 in der Kappe 29 verschlossen ist, der Reflektor 50 in der Form eines im Längsschnitt vorzugsweise parabelförmigen Kelchs angeordnet. Dieser Kelch ist weiter vorzugsweise auf der Platine 38 fixiert und wird bei Montieren der Platine 38 an der Kappe 29 in diese eingesetzt.

Der kelchförmige Reflektor 50 kann aus einem innenseitig verspiegeltem Kunststoffmaterial oder Aluminium (poliert und/oder verspiegelt bestehen. Alternativ ist es aber auch Möglich anstelle der Reflektors 50 einen Lichtleiter in Form eines Glaskörpers oder einem anderen Körper aus lichtleitendem Material zu verwenden, der vorzugsweise an seiner Mantelfläche eine Verspiegelung aufweist.

## Patentansprüche

1. Endoskop mit
- einer zumindest eine auf einer Platine (38) montierten Beleuchtungseinrichtung (36), sowie eine ein Linsensystem (34) und einen lichtempfindlichen Sensor (35) umfassende Optik (33) aufnehmenden Endkappe (29), welche die Platine (38) und die Optik (33) darin hält, wobei die Platine (38) den unteren Abschluss der Enkappe (29) bildet, die an der Stirnseite eines manuell abkrümmbaren Zwischenstücks (21) eines Endoskopschafts montiert ist und mit
- einer Kühleinrichtung für das Kühlen von der Platine getragenen elektrischen Bauteilen innerhalb der Kappe (29), vorzugsweise der Beleuchtungseinrichtung (36) bestehend aus einem ein Kühlmittel umfassenden Kühlkreislauf sowie einer Kühlkammer (24), die einen von der Endkappe (29) baulich getrennten Bestandteil des abkrümmbaren Zwischenstücks (21) bilden, wobei die Kühlkammer (24) durch die den unteren Abschluss der Kappe (29) bildende Platine (38) oder eine obere Abdeckplatte (25), an der die Platine (38) anliegt, abgedeckt wird, sodass bei Aufsetzen der Endkappe (29) auf das Zwischenstück (21) die Kühleinrichtung mit den zu kühlenden elektrischen Bauteilen oder mit diesen verbundenen Überbrückungsteilen in thermischen Kontakt bringbar ist.

2. Endoskop nach Anspruch 1, **dadurch gekennzeichnet, dass** die Kühleinrichtung eine Endplatte (20) des abkrümmbaren Zwischenstücks (21) bildet, in der die Kühlkammer (24) ausgeformt ist, in der entweder ein Wärmetauscher untergebracht ist oder die mit dem Kühlmittel geflutet wird, welches über Zu- und Ableitungen (40, 41) des Kühlkreislaufs innerhalb des abkrümmbaren Zwischenstücks (21) gefördert wird.

3. Endoskop nach Anspruch 2, **dadurch gekennzeichnet, dass** die Platine (38) an ihrer dem abkrümmbaren Zwischenstück (21) zugewandten Unterseite eine Anlagefläche aufweist, die mit der Endplatte (20) des abkrümmbaren Zwischenstücks oder Teile von dieser in Kontakt bringbar ist.

4. Endoskop nach Anspruch 2 oder 3, **dadurch gekennzeichnet, dass** die Endplatte (20) eine Becherform mit einem zentralen rohrstutzenförmigen Vorsprung (23) hat, wodurch eine ringförmige Rinne ausgebildet wird, welche die Kühlkammer bildet und die an einer Stirnseite der Endplatte (20) durch die Abdeckplatte (25) aus einem wärmeleitfähigen Material verschlossen ist.

5. Endoskop nach einem der vorstehenden Ansprüche **gekennzeichnet durch** ein im Längsschnitt kelchförmiger Reflektor (50) oder ein Lichtleiter aus Glas oder lichtdurchlässigem Kunststoff, der die Distanz zwischen der Beleuchtungseinrichtung (36) und einer Lichtaustrittsöffnung an der Vorderseite der Endkappe (29) überbrückt.

## Claims

1. An endoscope, comprising:
an end cap (29) accommodating at least a lighting means (36) mounted on an electric circuit board (38) and an optical means (33) comprising a lens system (34) and a light sensitive sensor (35) and holding the electric circuit board (38) and the optical means (33) therein, wherein the electric circuit board (38) forms an axially lower sealing cover of the end cap (29), the cap being mounted on the front of a manually bendable adapter (21) of an endoscope shaft; and
a cooling means for cooling electronic components arranged on the electric circuit board (38) inside the end cap (29), preferably the lighting means (36), wherein the cooling means comprises a cooling circuit feeding a cooling fluid and a cooling chamber (24), wherein the cooling circuit and the cooling chamber (24) form a separate part of the bendable adapter (21), wherein the cooling chamber (24) is covered by the electric circuit board (38) forming the lower sealing cover of the end cap (29), or an axially cover plate (25) that abuts the electric circuit board (38) so that upon placing the end cap (29) onto the adapter (21), the cooling means can be brought into thermal contact with the electronic components to be cooled or with bridging parts connected to the electronic components.

2. An endoscope according to claim 1, **characterized in that** the cooling means forms an end plate (20) of the bendable adapter (21), inside of which the cooling chamber (24) is formed, which accommodates a heat exchanger or is flooded with a cooling fluid delivered via feed (40) arid discharge pipes (41) inside the bendable adapter (21).

3. An endoscope according to claim 2, **characterized in that** the lower side of the electric circuit board (38) opposing the bendable adapter (21) constitutes an abutting surface that or parts thereof is engageable with the end plate (20) of the bendable adapter (21).

4. An endoscope according to claim 2 or 3, **characterized in that** the end plate (20) is cup-shaped having a central pipe-shaped projection (23) forming an annular groove, which forms the cooling chamber (24) and is closed at a front side of the end plate (20) by the cover plate (25) made of heat conducting material.

5. An endoscope according to any of the preceding claims, **characterized by** a reflector (50) that is goblet-shaped in longitudinal section, a light conductor made of glass or transparent plastic material for bridging the distance between the lighting means (36) and a light emerging opening at a front side of the end cap (29).

## Revendications

1. Endoscope doté
- d'un capuchon d'extrémité (29) recevant au moins un dispositif d'éclairage (36) monté sur une platine (38) et une optique (33) comprenant un système de lentille (34) et un capteur (35) sensible à la lumière, lequel capuchon maintient la platine (38) et l'optique (33) à l'intérieur, la platine (38) formant la fermeture inférieure du capuchon d'extrémité (29), lequel est monté sur le côté frontal d'une pièce intermédiaire (21), pouvant être recourbée manuellement, d'une tige d'endoscope et
- d'un dispositif de refroidissement pour le refroidissement de composants électriques portés par la platine à l'intérieur du capuchon (29), de préférence du dispositif d'éclairage (36) comprenant un circuit de refroidissement comprenant un réfrigérant et une chambre de refroidissement (24), qui forme un composant, séparé au niveau de la construction du capuchon d'extrémité (29), de la pièce intermédiaire (21) pouvant être recourbée, la chambre de refroidissement (24) étant recouverte par la platine (38) formant la fermeture inférieure du capuchon (29) ou une plaque de recouvrement (25) supérieure, sur laquelle s'applique la platine (38) de sorte que, lorsqu'on pose le capuchon d'extrémité (29) sur la pièce intermédiaire (21), le dispositif de refroidissement peut être mis en contact thermique avec les composants électriques à refroidir ou avec des parties de pontage reliées à ces composants.

2. Endoscope selon la revendication 1, **caractérisé en ce que** le dispositif de refroidissement forme une plaque d'extrémité (20) de la pièce intermédiaire (21) pouvant être recourbée, dans laquelle la chambre de refroidissement (24) est formée, dans laquelle un échangeur de chaleur est logé ou qui est inondé avec le réfrigérant, lequel est transporté par des conduites d'arrivée et d'évacuation (40, 41) du circuit de refroidissement à l'intérieur de la pièce intermédiaire (21) pouvant être recourbée.

3. Endoscope selon la revendication 2, **caractérisé en ce que** la platine (38) présente sur son côté inférieur, tourné vers la pièce intermédiaire (21) pouvant être recourbée, une surface d'appui qui peut être mise en contact avec la plaque d'extrémité (20) de la pièce intermédiaire pouvant être recourbée ou des parties de celle-ci.

4. Endoscope selon la revendication 2 ou 3, **caractérisé en ce que** la plaque d'extrémité (20) a une forme de gobelet avec une saillie (23) centrale, en forme de tubulure, de sorte qu'une goulotte de forme annulaire est réalisée, laquelle forme la chambre de refroidissement et qui est fermée sur un côté frontal de la plaque d'extrémité (20) par la plaque de recouvrement (25) à base d'un matériau thermoconducteur.

5. Endoscope selon l'une des revendications précédentes, **caractérisé par** un réflecteur (50) en forme de calice en coupe longitudinale ou un conducteur de lumière en verre ou en plastique transparent, qui franchit la distance entre le dispositif d'éclairage (36) et une ouverture de sortie de lumière sur le côté frontal du capuchon d'extrémité (29).
